# EUROPEAN PATENT APPLICATION

(11) **EP 4 483 802 A1**
(43) Date of publication of application: **01.01.2025**
(21) Application number: 24185677.2
(22) Date of filing: 01.07.2024
(51) Int. Cl.: A61B 5/06, A61B 5/287, A61B 5/00, A61B 18/14, A61B 34/20, A61B 18/00

(54) **SYSTEM FOR SHAPE TRACKING OF INTRABODY OBJECT SUBJECT TO DEFORMATION**

(30) Priority: 29.06.2023 US 202363524144 P; 28.06.2024 US 202418758437
(71) Applicant: Biosense Webster (Israel) Ltd., 2066717 Yokneam (IL)
(72) Inventor: BAR-TAL, Meir, 2066717 Yokneam (IL); VAN NIEKERK, Pieter, Irvine, 92618 (US); RODRIGUEZ SOTO, Juan, Irvine, 92618 (US); BASU, Shubhayu, Irvine, 92618 (US); OSADCHY, Daniel, 2066717 Yokneam (IL); EBRAHIMI, Babak, Irvine, 92618 (US)
(74) Representative: Carpmaels & Ransford LLP

(57) **Abstract**

An intrabody probe with a flexible portion is configured with one or more integrated EM loops of electrically-conductive elements wherein the EM loops when exposed to one or more external magnetic fields are configured to generate voltage signals that are indicative of position of the EM loops, such as when the EM loops are subjected to an external force that results in deformation. With multiple EM loops at different locations on the flexible portion of the probe, a collection of such voltage signals from each EM loop enables a system configured to receive and process the voltage signals to determine coordinates of the EM loops in a local coordinate system and track a shape in space representative of probe deformation in real time.

## Description

### PRIORITY

This application claims the benefit of U.S. Patent Application 63/524,144, entitled "System And Method For Shape Tracking Of Intrabody Object Subject To Deformation", filed June 29, 2023, the disclosure of which is incorporated herein by reference.

### FIELD OF INVENTION

This invention relates generally to detecting location of a flexible object placed within a living body, and specifically to sensing multiple conductive elements on a flexible electrophysiology probe or device so as to track its shape and deformation in space in real-time.

### BACKGROUND

Tracking the position of intrabody objects, such as sensors, tubes, catheters, dispensing devices, and implants, is required for many medical procedures. Well-established, highly accurate systems for determining the position and orientation of an intrabody object have been developed based on magnetic field sensing. These systems utilize sensors affixed to the intrabody object to measure the relative strengths of externally-generated magnetic fields and to derive from these measurements the position of the object. Methods for magnetic based position sensing are disclosed, for example, in U.S. Patent Nos. 5,391,199, 5,433,489, and 6,788,967 to Ben-Haim, in U.S. Patent No. 6,690,963 to Ben-Haim, et al., in U.S. Patent No. 5,558,091 to Acker et al., in U.S. Patent No. 6,172,499 to Ashe, and in U.S. Patent No. 6,177,792 to Govari, all of which disclosures are incorporated herein by reference.

Position sensing systems have also been developed which utilize impedance-based measurements. In such systems, impedance is measured between electrodes fixed to the intrabody object and electrodes placed on the body surface. The systems then derive the position of the intrabody object from the impedance measurements. Methods for impedance-based position sensing are disclosed, for example, in U.S. Patent No. 5,983,126 to Wittkampf, in U.S. Patent No. 6,456,864 to Swanson, and in U.S. Patent No. 5,944,022 to Nardella, all of which disclosures are incorporated herein by reference.

Hybrid position sensing systems and methods which combine magnetic and electrical position sensing techniques are disclosed, for example, in U.S. Patent Publication No. 20070016007 to Govari. In these systems, a magnetic position sensor provides an accurate position reference for calibrating less accurate, electrical impedance-based measurements. For this purpose, a hybrid probe, such as a catheter, comprising a triaxial magnetic position sensor and one or more electrodes is used to correlate the magnetic position measurements with the impedance-based measurements. Systems of this sort alleviate the need for multiple magnetic position sensors, and thus benefit from both magnetic position sensing and impedance-based sensing.

However, for catheters with relatively large distal end assemblies, especially those that present a 3-D configuration, magnetic, impedance-based or hybrid position sensing provides only "point" location of the corresponding sensor and not of the 3-D configuration as a whole, especially as a structure with segments connected by joints. Moreover, because magnetic position sensors, whether single axis or multi-axes, are rigid in structure, they are not suitable for mounting on flexible portions of catheters to detect flexion or deformation, including distal end assemblies that are flexible or those that bend at an interface with a shaft. Because magnetic position sensors are mounted on rigid portions of catheter shafts, they do not sense the change in position of distal assemblies due to bend. As such, magnetic position sensors are not typically used to detect deformations in distal assemblies, for example, due to bending or flexion when pressed against the wall of the heart chamber.

Accordingly, applicants recognized that there is a need to provide a system and method that can sense and track a shape or volume of an intrabody device, for example, an electrophysiology catheter, especially with a 3-D distal assembly, in space and its shape and deformation and/or the deformation in real-time when the distal assembly is subjected to an external force such as when the distal assembly comes into contact with tissue.

### SUMMARY OF THE DISCLOSURE

An intrabody flexible probe adapted to deform when subjected to an external force includes one or more integrated electromagnetic (EM) loops of electrically-conductive elements that are responsive to one or more magnetic fields to provide electrical signals indicative of the position the probe and its shape in space including its deformation in real-time. The EM loops that may be carried on or embedded in the flexible distal assembly and/or the flexible shaft of the probe are configured to generate voltage signals in response to magnetic fluxes that induce currents through the EM loops. When the configuration of an EM loop changes due to deformation, the voltage signal of the EM loop correspondingly changes in response to the deformation. With a sufficient plurality of EM loops positioned at different locations of the flexible portion(s) of the probe, a collection of such responsive voltage signals may enable a system configured to receive and process the voltage signals to determine location coordinates of the EM loops in a local coordinate system and track a shape representative of the flexible probe in real-time.

In some embodiments, an intrabody probe comprises a deformable portion; and a flexible electromagnetic loop positioned on the deformable portion, the flexible electromagnetic loop configured to generate an induced voltage in response to an external magnetic field.

In some embodiments, the intrabody probe further comprises a distal electrode assembly and the deformable portion includes a deformable portion of the distal electrode assembly.

In some embodiments, the intrabody probe further comprises a shaft and the deformation portion includes a portion of a shaft.

In some embodiments, the intrabody probe further comprises a distal electrode assembly, a shaft, and an interface therebetween, and the deformation includes a portion of the interface.

In some embodiments, the flexible electromagnetic loop includes an electrically conductive spine.

In some embodiments, the flexible electromagnetic loop includes an electrically-conductive coating.

In some embodiments, the flexible electromagnetic loop includes an electrical trace.

In some embodiments, the flexible electromagnetic loop includes a flex circuit.

In some embodiments, the flexible electromagnetic loop is configured to generate a different induced voltage when the deformable portion experiences a deformation that changes the flexible magnetic loop from a neutral configuration into a deformed configuration.

In some embodiments, the induced voltage is indicative of a location of the flexible electromagnetic loop.

In some embodiments, the flexible electromagnetic loop is configured to generate the induced voltage in response to a first external magnetic field at a first frequency and to generate a second induced voltage in response to a second external magnetic field at a second frequency.

In some embodiments, an intrabody probe comprises a deformable portion, and a plurality of flexible electromagnetic loops, each in a different position on the deformable portion, each flexible electromagnetic loop configured to generate a respective induced voltage in response to an external magnetic field.

In some embodiments, at least one of the flexible electromagnetic loop includes an electrically conductive spine.

In some embodiments, at least one of the flexible electromagnetic loop includes an electrically-conductive coating.

In some embodiments, at least one of the flexible electromagnetic loop includes an electrical trace.

In some embodiments, at least one of the flexible electromagnetic loop includes a flex circuit.

In some embodiments, the flexible electromagnetic loops are configured to generate induced voltages indicative of locations of the loops in a local coordinate system.

In some embodiments, the flexible electromagnetic loops are configured to generate induced voltages indicative of deformations of the loops in a local coordinate system when the deformable portion transitions from a neutral configuration to a deformed configuration.

In some embodiments, each of the plurality of flexible electromagnetic loops is configured to generate the respective induced voltage in response to a first external magnetic field at a first frequency and to generate a respective second induced voltage in response to a second external magnetic field at a second frequency.

In some embodiments, each of the plurality of flexible electromagnetic loops is configured to generate a respective induced voltage when subjected to an external force that causes deformation of the respective loop, and a collection of the respective induced voltages of the plurality of loops is representative of a deformed shape of the deformable portion.

In some embodiments, an intrabody probe comprises a deformable portion, and a first electrically-conductive element and a second electrically-conductive element, the first and second electrically-conductive elements configured as a flexible electromagnetic loop on the deformation portion. The intrabody probe further comprises a first terminal of the first electrically-conductive element configured to carry a first induced voltage when exposed to a magnetic field, a second terminal of the second electrically-conductive element configured to carry a second induced voltage when exposed to the magnetic field, and a connection point configured to provide a voltage difference between the first and second induced voltages.

In some embodiments, the probe includes an electrophysiology catheter with a flexible end effector, the flexible end effector including the deformable portion.

In some embodiments, the probe includes an electrophysiology catheter with a flexible shaft, the flexible shaft including the deformable portion.

In some embodiments, the end effector includes first and second splines configured as the first and second electrically-conductive elements.

In some embodiments, the first and second electrically-conductive elements include first and second electrically-conductive coatings on the end effector.

In some embodiments, the intrabody probe further comprises a flexible printed circuit board affixed to the end effector, the printed circuit board including the first and second electronically-conductive elements.

In some embodiments, the first and second electrically-conductive elements are affixed to an outer surface of the shaft.

In some embodiments, the shaft includes a sidewall and the first and second electrically-conductive elements include first and second wires embedded in the sidewall.

In some embodiments, the first electrically-conductive element includes a nonlinear segment.

In some embodiments, at least the first electrically-conductive element extends circumferentially about the shaft.

In some embodiments, a system comprises a magnetic field generator configured to generate a magnetic field, and a catheter. The catheter comprises a deformable portion, a plurality of flexible electromagnetic loops, and at least a connection point. Each of the plurality of flexible electromagnetic loops is affixed to a different position on the deformation portion, with each loop including a first electrically-conductive element, a second electrically-conductive element, a first terminal of the first electrically-conductive element configured to carry a first induced voltage when exposed to the magnetic field, and a second terminal of the second electrically-conductive element configured to carry a second induced voltage when exposed to the magnetic field. The at least a connection point is configured to provide a voltage difference between the first and second induced voltages of each loop. The system further comprises a display, and a processor that is configured to (i) receive the voltage difference of each loop from the at least one connection point; (ii) determine coordinates of each loop relative to a local coordinate system; and (iii) drive the display to display a shape as a visual representation of the deformable portion based on a collection of the coordinates of each loop.

In some embodiments, the deformable portion includes electrodes, and the system further includes an RF generator configured to energize the electrodes for ablation of tissue.

In some embodiments, the deformable portion includes a flexible end effector.

In some embodiments, the deformable portion includes a flexible shaft.

In some embodiments, the deformable portion includes an end effector with first and second splines that are configured as the first and second electrically-conductive elements.

In some embodiments, the first and second electrically-conductive elements include first and second electrically-conductive coatings on the end effector.

In some embodiments, the system further comprises a flexible printed circuit board affixed to the end effector and the printed circuit board includes the first and second electronically-conductive elements.

In some embodiments, the first and second electrically-conductive elements are affixed to an outer surface of the shaft.

In some embodiments, the shaft includes a sidewall and the first and second electrically-conductive elements include first and second wires embedded in the sidewall.

In some embodiments, the display displays the shape as a visual representation in real-time.

In some embodiments, a system comprises a magnetic field generator configured to generate a magnetic field, and a catheter. The catheter comprises a deformable portion, a plurality of flexible electromagnetic loops, and at least a first connection point. Each of the plurality of flexible electromagnetic loops is affixed to the deformation portion at a different position, each loop including a first electrically-conductive element, a second electrically-conductive element, a first terminal of the first electrically-conductive element configured to carry a first induced voltage when exposed to the magnetic field, and a second terminal of the second electrically-conductive element configured to carry a second induced voltage when exposed to the magnetic field. The at least a connection point is configured to provide a voltage difference between the first and second induced voltages of each loop. The system further comprises a display, and a processor that is configured to (i) receive the voltage difference of each loop from the at least one connection point; (ii) determine coordinates of each loop relative to a local coordinate system; (iii) identify a shape for the deformable portion based on a collection of the coordinates of each loop; and (iv) drive the display to display the shape as a visual representation of the deformable portion.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 is a schematic illustration of a system, in accordance with implementations of the disclosed subject matter.
FIG. 2A is a perspective view of an intrabody probe for use with the system of FIG. 1, in a first configuration prior to deployment from a guiding sheath, in accordance with implementations of the disclosed subject matter.
FIG. 2B is a perspective view of the intrabody probe of FIG. 2A, in a second configuration after deployment from the guiding sheath.
FIG. 3 is a top plan view of an end effector of the intrabody probe of FIG. 2B, in a neutral configuration, in accordance with implementations of the disclosed subject matter.
FIG. 4 is a top plan view of an end effector in a neutral configuration, in accordance with alternate implementations of the disclosed subject matter.
FIG. 5 is a front perspective view of the end effector of FIG. 2B, in a deformed configuration when subjected to an external force.
FIG. 6 is a side view of a basket end effector, in a neutral configuration, in accordance with implementations of the disclosed subject matter.
FIG. 7 is a side view of a balloon end effector, in a neutral configuration, in accordance with implementations of the disclosed subject matter.
FIG. 8A is a front perspective view of a balloon catheter in accordance with implementations of the disclosed subject matter, with a balloon end effector.
FIG. 8B is a top plan view of a first substrate of a PCB of the balloon end effector of FIG. 8A.
FIG. 8C is a top plan view of a second substrate of a PCB of the balloon end effector of FIG. 8A.
FIG. 9A is a side elevational view of a shaft with an EM loop, in accordance with a first implementation of the disclosed subject matter, in a neutral configuration.
FIG. 9B is a side elevational view of a shaft with an EM loop, in accordance with a second implementation of the disclosed subject matter, in a deflected configuration.
FIG. 9C is a side elevational view of a shaft with an EM loop, in accordance with a third implementation of the disclosed subject matter, in a deflected configuration.
FIG. 9D is a side elevational view of a shaft with an EM loop, in accordance with a fourth implementation of the disclosed subject matter, in a neutral configuration.

### DETAILED DESCRIPTION OF THE EMBODIMENTS

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitations, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

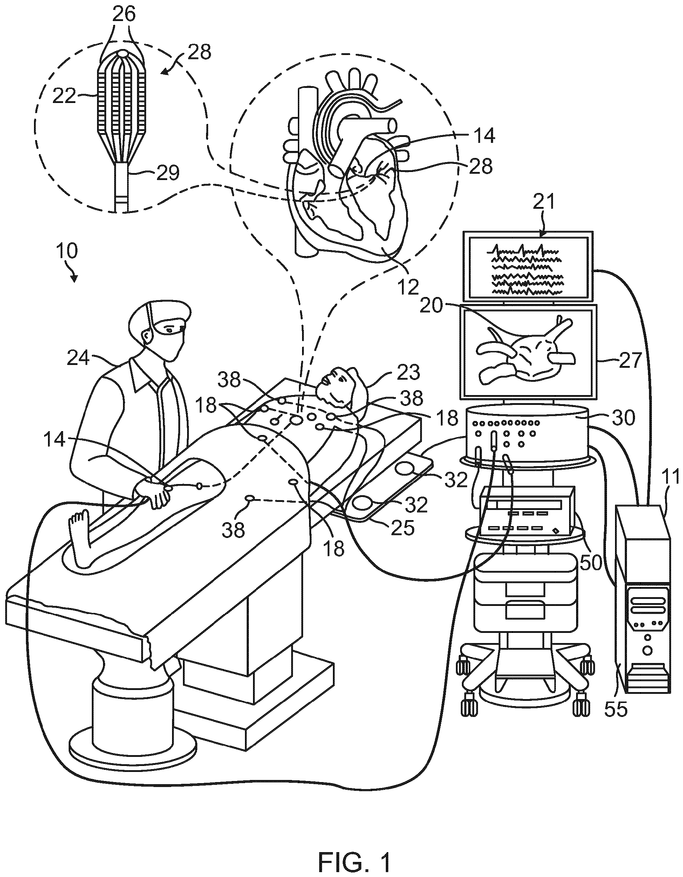

Reference is made to FIG. 1 showing an example catheter-based electrophysiology mapping and ablation system (10). System (10) includes multiple catheters, which are percutaneously inserted by a physician (24) through the patient's vascular system into a chamber or vascular structure of a heart (12). Typically, a delivery sheath catheter is inserted into the left or right atrium near a desired location in heart (12). Thereafter, one or more catheters may be inserted into the delivery sheath catheter so as to arrive at the desired location in heart (12). The plurality of catheters may include catheters dedicated for sensing Intracardiac Electrogram (IEGM) signals, catheters dedicated for ablating and/or catheters dedicated for both sensing and ablating. An example catheter (14) that is configured for sensing IEGM is illustrated herein. Physician (24) may place a distal tip (28) of catheter (14) in contact with the heart wall for sensing a target site in heart (12). For ablation, physician (24) may similarly place a distal end of an ablation catheter in contact with a target site for ablating tissue.

Catheter (14) is an exemplary catheter that includes one and preferably multiple electrodes (26) optionally distributed over a plurality of splines (22) at distal tip (28) and configured to sense the IEGM signals. Catheter (14) may additionally include a position sensor (29) embedded in or near distal tip (28) for tracking position and orientation of distal tip (28). Optionally and preferably, position sensor (29) is a magnetic based position sensor including three magnetic coils for sensing three-dimensional (3D) position and orientation.

Magnetic based position sensor (29) may be operated together with a location pad (25) including a plurality of magnetic coils (32) configured to generate magnetic fields in a predefined working volume. Real time position of distal tip (28) of catheter (14) may be tracked based on magnetic fields generated with location pad (25) and sensed by magnetic based position sensor (29). Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System (10) includes one or more electrode patches (38) positioned for skin contact on patient (23) to establish location reference for location pad (25) as well as impedance-based tracking of electrodes (26). For impedance-based tracking, electrical current is directed to electrodes (26) and sensed at electrode skin patches (38) so that the location of each electrode can be triangulated via the electrode patches (38). Details of the impedance-based location tracking technology are described in US Patent Nos. 7,536,218; 7,756,576; 7,848,787; 7,869,865; and 8,456,182.

A recorder (11) records and displays electrograms (21) captured with body surface ECG electrodes (18) and intracardiac electrograms (IEGM) captured with electrodes (26) of catheter (14). Recorder (11) may include pacing capability for pacing the heart rhythm and/or may be electrically connected to a standalone pacer.

System (10) may include an ablation energy generator (50) that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) (30) is an interface configured to establish electrical communication between catheters, other electrophysiological equipment, power supply and a workstation (55) for controlling operation of system (10). Electrophysiological equipment of system (10) may include for example, multiple catheters, location pad (25), body surface ECG electrodes (18), electrode patches (38), ablation energy generator (50), and recorder (11). Optionally and preferably, PIU (30) additionally includes processing capability for implementing real-time computations of location of the catheters and for performing ECG calculations.

Workstation (55) includes memory, processor unit with memory or storage with appropriate operating software stored therein, and user interface capability. Workstation (55) may provide multiple functions, optionally including (1) modeling the endocardial anatomy in three-dimensions (3D) and rendering the model or anatomical map (20) for display on a display device 27, (2) displaying on display device (27) activation sequences (or other data) compiled from recorded electrograms (21) in representative visual indicia or imagery superimposed on the rendered anatomical map (20), (3) displaying real-time location and orientation of multiple catheters within the heart chamber, and (4) displaying on display device (27) sites of interest such as places where ablation energy has been applied. One commercial product embodying elements of the system (10) is available as the CARTOTM 3 System, available from Biosense Webster, Inc., 31A Technology Drive, Irvine, CA 92618.

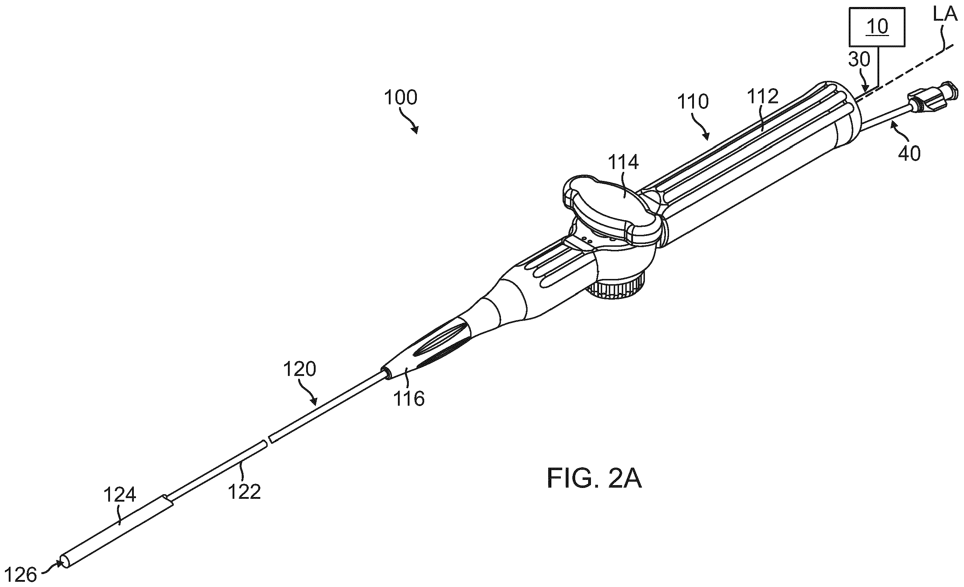

As shown in **FIGS. 2A-2B****,** a catheter assembly (100) includes handle assembly (110), catheter (120) extending distally from handle assembly (110), end effector (200) located at a distal end of catheter (120), and a deflection drive actuator (114) associated with handle assembly (110). Deflection drive actuator (114) is rotatable relative to a casing (112) of handle assembly (110) to thereby deflect end effector (200) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

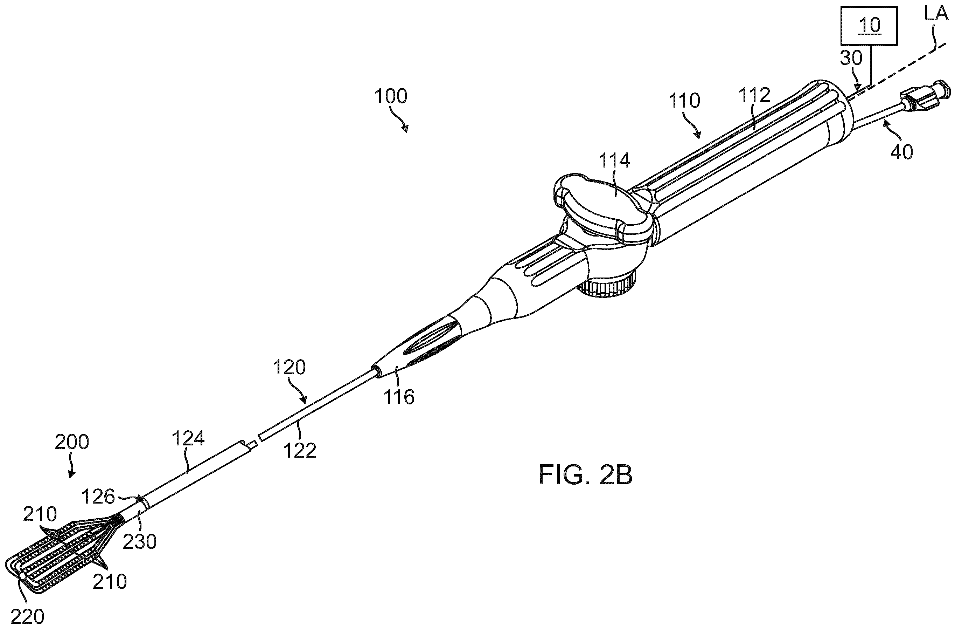

Catheter (120) includes an elongate flexible shaft (122) and an outer sheath (124). Shaft (122) is coaxially and slidably disposed within outer sheath (124). End effector (200) is positioned at the distal end of shaft (122). The proximal end of catheter (120) extends distally from a nozzle member (116) of handle assembly (110). In some versions, outer sheath (124) is an integral component of catheter (120). In some other versions, sheath (124) is a component of another instrument, and catheter (120) is inserted into sheath (124). In either scenario, shaft (122) and an outer sheath (124) may transition between two arrangements, including a first arrangement as shown in **FIG. 2A** where outer sheath (124) is distally positioned in relation to shaft (122), such that end effector (200) is contained within outer sheath (124); and a second arrangement as shown in **FIG. 2B** where outer sheath (124) is proximally positioned in relation to shaft (122), such that end effector (200) is exposed relative to outer sheath (124). In some versions, shaft (122) translates relative to handle assembly (110) to achieve the different longitudinal positioning relative to outer sheath (124). In some other versions, outer sheath (124) translates relative to handle assembly (110) to achieve the different longitudinal positioning relative to shaft (122).

Catheter (120) and end effector (200) may be in the state shown in **FIG. 2A** when catheter (120) is introduced into the body of the patient (PA); and during transit from the insertion site to the targeted cardiovascular region within the patient (PA). Once catheter (120) and end effector (200) reach the targeted cardiovascular region within the patient (PA), outer sheath (124) may be retracted proximally to expose end effector (200), thereby allowing end effector (200) to achieve the expanded, deployed state shown in **FIG. 2B**

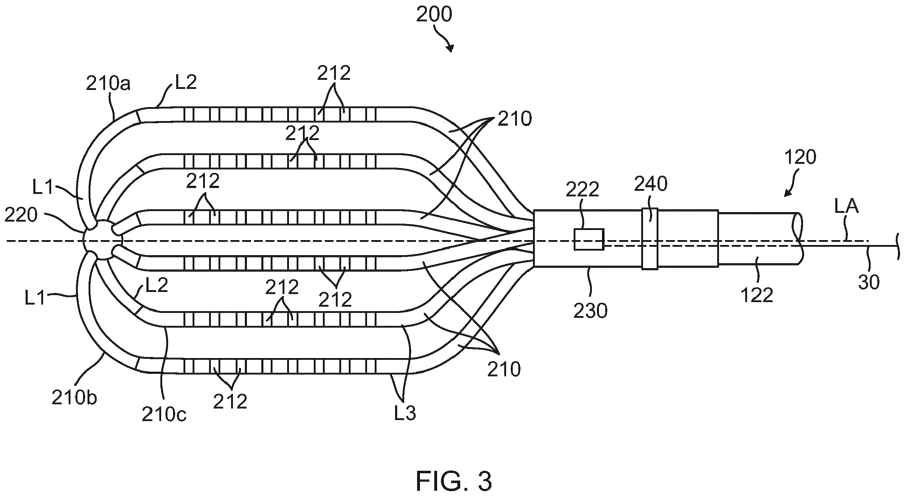

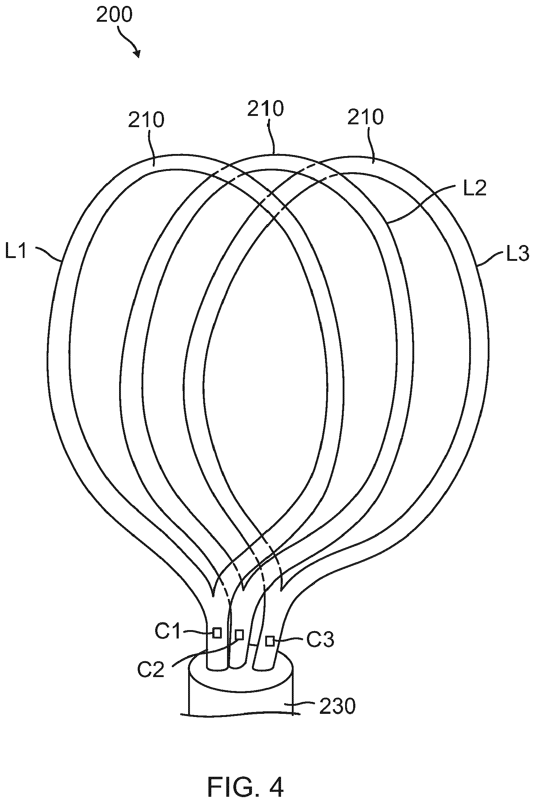

As shown in **FIG. 2B** and **FIG. 3****,** end effector (200) is secured to the distal end of shaft (122) of catheter (120) via a proximal coupling member (230). End effector (200) of the present example includes a plurality of splines (210) that extend distally from the proximal connector 230. As an interface 213 with the proximal connector 230, the proximal portion of each spline (210) extends distally and diverges outwardly away from the central longitudinal axis (LA) defined by a proximal portion of catheter (120). The longitudinally intermediate portion of each spline (210) is generally parallel with the central longitudinal axis (LA). The distal portion of each spline converges back toward the central longitudinal axis (LA). For example, a distal end of each spline is distally joined to a distal coupling (220) in the form of a ball **(****FIG. 3****),** or alternatively, each spline can be formed from a linear spline that is bent into a U-shape such that the distal end and the proximal end converges at the proximal connector 230 **(****FIG. 4****).** Each spline (210) may also include a plurality of electrodes (212) that are spaced apart from each other along the length of spine (210) **(****FIG. 3****).** In some versions, electrodes (212) are operable to provide EP signals from tissue for EP mapping. In addition, or in the alternative, electrodes (212) may be operable to ablate tissue. Splines (210) may also include various other features, such as temperature sensors, etc.

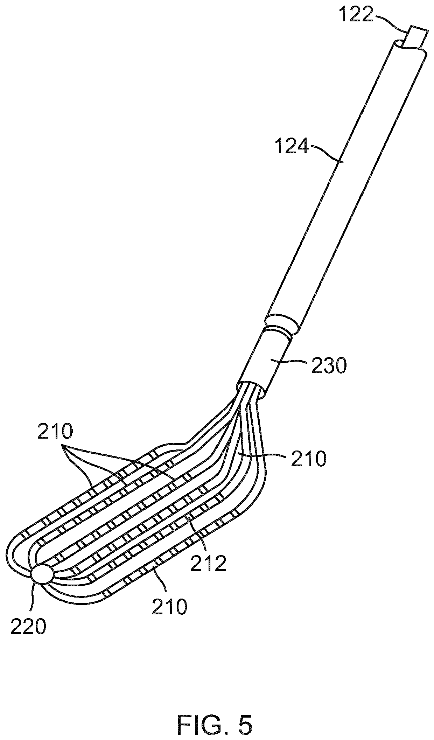

Splines (210) may be formed of a resilient material (e.g., nitinol, etc.) such that splines (210) may be resiliently biased to assume the configuration shown in **FIG. 2B****,** **FIG. 3** and **FIG. 4****.** In some versions, end effector (200) defines a generally planar configuration when splines (210) are in the state shown, with the plane defined by end effector (200) extending along and transversely from the longitudinal axis (LA). Splines are also deformable such that splines may be contained within outer sheath (124) as shown in **FIG. 2A****.** Splines may thus deform inwardly toward the longitudinal axis (LA), along the plane defined by end effector (200), to fit within sheath (124). In addition to deforming and expanding along the plane defined by end effector (200) to transition between the state shown in **FIG. 2A** and the state shown in **FIG. 2B** and **FIG. 3****,** splines may also deform along a direction that is transverse to the plane defined by end effector (200). Such deformation may occur as end effector (200) is pressed against tissue (**FIG. 5****).**

The proximal coupling member (230) may take any suitable form. In some embodiments, the coupling member (230) comprise a cylindrical plastic (e.g., polyether ether ketone (PEEK), etc.) component. In some embodiments, the cylindrical plastic is flexible and deformable. The proximal ends of spines (210) may be fixedly secured to coupling member (230) via adhesive, overmolding, welding, epoxy, or in any other suitable fashion. Similarly, the distal end of flexible shaft (122) may be fixedly secured to coupling member (230) via adhesive, overmolding, welding, epoxy, or in any other suitable fashion. In some versions, the distal end of flexible shaft (122) is fixedly secured to the proximal ends (and distal ends in some embodiments) of splines (210) within coupling member (230), in addition to these components being fixedly secured to coupling member (230). Coupling member (230) of the present example also includes a ring electrode (240). In some versions, ring electrode (240) is used to provide a reference signal from blood of a patient while electrodes (210) provide EP mapping signals from tissue of the patient. Alternatively, ring electrode (240) may be used for any other suitable purpose(s). In some variations, ring electrode (240) is omitted.

In some versions, the workstation 55 (FIG. 1) is also operable to receive EM loop signals indicative of position and/or shape from the catheter assembly (100), as will be described in greater detail below. In such versions, the workstation 55 is also operable to process the EM loop signals, including voltage signals, to thereby determine the position and/or shape of end effector (200) or other components of catheter assembly (100) within the patient (PA). Location pad (25) may include coils that generate alternating magnetic fields in a predetermined working volume that contains the heart (H). Each generator may also drive the coils at different frequencies.

Display device (27) is coupled with the workstation (55) and is operable to render images of patient anatomy. Such images may be based on a set of preoperatively or intraoperatively obtained images (e.g., a CT or MRI scan, 3-D map, etc.). The views of patient anatomy provided through display device (27) may also change dynamically based on signals indicative of position and/or shape of catheter assembly (100), as described further below. For instance, as end effector (200) of catheter (120) moves within the patient (PA), corresponding position and/or shape data from catheter may cause the workstation (55) to update the patient anatomy views in display device (27) in real time to depict the regions of patient anatomy around end effector (200) as end effector (200) moves within the patient (PA). Moreover, the workstation (55) may drive display device (27) to show locations of aberrant conductive tissue sites, as detected via EP mapping with end effector (200). By way of example only, the workstation (55) may drive display device (27) to superimpose the locations of aberrant conductive tissue sites on the images of the patient's anatomy, such as by superimposing an illuminated dot, a crosshair, or some other form of visual indication of aberrant conductive tissue sites.

The workstation (55) may also advantageously drive display device (27) to superimpose the current position and/or shape of end effector (200), the shaft (122), including any deformation experience by the catheter (100) of its flexible portion, on the images of the patient's anatomy, such as by superimposing a graphical representation of such deformation. Such a superimposed visual indication may also move within the images of the patient anatomy on display device (27) in real time as the physician moves end effector (200) within the patient (PA), thereby providing real-time visual feedback to the operator about the position and/or shape of the catheter (100) within the patient (PA) as end effector (200) moves within the patient (PA). The images provided through display device (27) may thus effectively provide a video tracking the position and/or shape of the catheter and its deformation within the patient (PA), without necessarily having any optical instrumentation (i.e., cameras) viewing end effector (200). In the same view, display device (27) may simultaneously visually indicate the locations of aberrant conductive tissue sites detected through the EP mapping. The physician (PH) may thus view display device (27) to observe the real time positioning and/or shape of the catheter elements in relation to the mapped aberrant conductive tissue sites and in relation to images of the adjacent anatomical structures in the patient (PA).

The present example may include a fluid source of the present example includes a bag containing saline or some other suitable irrigation fluid. Conduit includes a flexible tube that is further coupled with a pump, which is operable to selectively drive fluid from fluid source to catheter assembly. In some variations, conduit, fluid source, and pump are omitted entirely. In versions where these components are included, end effector may be configured to communicate irrigation fluid from fluid source to the target site in the patient. Such irrigation may be provided in accordance with the teachings of any of the various patent references cited herein; or in any other suitable fashion as will be apparent to those skilled in the art in view of the teachings herein.

The embodiments of the catheters shown in **FIG.3** and **FIG. 4** include one or more electromagnetic (EM) loops of electrically-conductive material that are formed on or otherwise integrated into one or more flexible portions of the catheter which can deform when subjected to an external force. For example, as shown in **FIG. 4****,** extending from a distal end of the shaft are splines (210) that form closed EM spline loops (L1, L2, L3). Each spline loop is formed from a linear spline that is curved or bent upon itself such that its distal end converges with its proximal end at the interface (213) with the distal end of the shaft. Each of the spline loop occupies a different position on the end effector (200) and each loop may generate a voltage signal based on the magnetic field flux through the respective loop at the location of the respective loop. The voltage reading for each loop may be provided at respective connection points (C1, C2, C3).

According to implementations of the disclosed subject matter, each EM loop is subjected to one or more alternating magnetic fields around the heart (H) of the patient (PA) generated by one or more field generators (32) of location pad (25). Each loop may generate a voltage amount based on a magnetic field at the location of the loop. The console (12) with a processor receives voltage signals generated by each EM loops in response to the magnetic flux of at least one alternating magnetic field, where the voltage signals may be measured from a voltage contact that is part of the loop or external to the loop. With multiple magnetic fields that are, for example, generated at different frequencies, each magnetic field can induce a respective voltage in each loop such that the processor can ascertain position and orientation of each loop in a defined 3-D coordinate system where a collection of such respective voltages of loops in different positions on the catheter.

The processor may construct an electromagnetic map of the heart, based on any ECG signals (which indicate electrical activity of heart tissue) and the voltages induced in the loops (which indicate the respective locations of the loops and the sources of the ECG signals). Such a map may be displayed on the display 18.

Advantageously, when a flexible portion of a catheter is subjected to an external force that causes deformation, the loops in the flexible portion experience deformations as they transition from neutral configurations to deformed configurations, where such transitions changes their respective induced voltages as a result of changes in the respective magnetic fluxes. With reference to FIG. 5, the catheter 100 has been maneuvered into contact with tissue such that the end effector 200 is no longer parallel with the longitudinal axis LA defined by the shaft 122 but extending at an angle with deformation at, for example, the interface 213 which transitions from a neutral configuration, as shown in FIG. 2B, to a deformed or bent configuration, as shown in FIG. 5. And with each spline loop L1, L2, L3 occupying a respective position on the end effector 200, each spline loop may undergo a deformation that impacts and alters the magnetic flux detected by each loop thereby changing the respective voltage signal induced by the magnetic fields.

When the processor of the console (12) detects changes in the voltage signals of the spline loops L1, L2, L3, these changes indicate the spline loops have been deformed as a result of the external force causing deformation of the interface 213. It is understood that for a flexible end effector of any configuration, whether 2-D or 3-D, coverage of the structure of the end effector with a sufficient plurality of EM loops on the surface and/or in the interior of the end effector, each in a different position or location on the end effector, enables the processor to monitor and track not only the position and orientation but the overall shape of the end effector and any deformation it undergoes in a space defined by a local coordinate system.

Where the multiple alternating magnetic fields are generating magnetic fluxes at different frequencies, the voltage signals (and changes thereof) due to movement and deformation of the loops are measured at the respective frequencies to provide signals that are indicative of such movement and deformation.

It can be seen from the embodiment of the end effector (200) of **FIG. 3** that each spline (210) (either as having a construction of an electrically-conductive material or serving as a substrate on to which an electrically-conductive material is applied or integrated into) can form a loop with any other spline. For example, spline (210a) may form a first loop (L1) via the distal coupling (220) with spline (210b), and spline (210a) may form a second loop (L2) via the distal coupling (220) with spline (210c), and spline (210b) and spline (210c) may form a third loop (L3) via the distal coupling (220). It is understood that a myriad of different loops can be created and designated from coupling different splines, where each of these loops may generate a distinct voltage signal based on the magnetic fields, generated by the field generators (32) of location pad (25), at the location of the end effector (200), with voltage readings provided at voltage measurement area or connection point (222). Therefore, depending on the location of the splines (or the electrically-conductive elements thereon) on the end effector (200) and the designation and identity of the splines used to create the loops, the guidance and drive system 10 is able to detect the location of the loops (and hence of the catheter 100), along with the shape of the end effector and any deformation of that shape of the catheter, including any deformation of the end effector, the shaft, and/or the interface by the voltages measured at the connection point (222). To that end, one or more connection points 22 may be a voltage contact, such as a conductive pad or a wired connection, that is coupled to the guidance and drive system (10) via cable 30 through which the loop voltage signals are received and processed by the first driver module (14).

At a connection point, of the pair of first and second splines that generally converge to form a loop a first terminal of a first conducting element carries a first voltage induced by the magnetic field(s) through the loop and a second terminal of a second conducting element carries a second voltage induced by the magnetic field through the loop. The pair of first and second conducting elements collectively carry to a differential amplifier (not shown) the voltage difference that is induced across the first and second conducting elements forming the loop. The outputs from the amplifier are representative of position and/or shape of the loop. It is understood that depending on the loops of interest, the voltage difference may be measured for any plurality of splines (and their respective conducting elements) that form the loops.

The processor of the console (12) is operable to process the loop voltage signals to thereby determine the position of each loop, and the deformation of each loop, and therefore, collectively, the shape of the end effector and shaft on which the loops reside. With multiple loops configured on different locations on the end effector and the interface with the shaft, the multiple loops collectively connect the overall structure of the end effector and the interface. Advantageously, the more loops configured at different locations on the catheter, the more complete the system can track position and the full shape of the catheter in space.

Coupled to the processor of the console (12), display device (27) of **FIG.** 1 can render images of patient anatomy based on a set of preoperatively or intraoperatively obtained images (e.g., a CT or MRI scan, 3-D map, etc.) The views of patient anatomy provided through display device (27) may also change dynamically based voltage signals from the loops of the catheter (100). For instance, as the end effector of FIG. 5 moves and/or deforms within the patient, the corresponding voltage signals may cause the processor of the console (12) to update the patient anatomy views in display device (27) in real time to depict the regions of patient anatomy around the end effector as the end effector moves or deforms within the patient. Moreover, the workstation (55) may drive display device (27) to show locations of aberrant conductive tissue sites, as detected via electrophysiological (EP) mapping with the electrodes 212 on the end effector (200) or as otherwise detected (e.g., using a dedicated EP mapping catheter, etc.).

The workstation (55) may also drive display device (27) to superimpose the current location of the end effector, along with its shape, on the images of the patient's anatomy, such as by superimposing an illuminated graphic representative of the shape or some other form of visual indication. Such a superimposed visual indication may also move within in the images of the patient anatomy on display device (27) in real time as the physician moves the end effector within the patient (PA), thereby providing real-time visual feedback to the operator about the position and shape of the end effector within the patient (PA) as the end effector moves within the patient, especially where the end effector is advanced into tissue wall which imparts an external force on the end effector to deform and change the shape of the end effector. Where the splines are located in the region(s) of the end effector or the shaft that deform as a result of the external force, the deformation(s) are also displayed in real-time. The images provided through display device (27) may thus effectively provide a video tracking the position and shape of the end effector within a patient (PA), without necessarily having any optical instrumentation (i.e., cameras) viewing the end effector. In the same view, the display device (27) may simultaneously visually indicate the locations of aberrant conductive tissue sites detected through EP mapping. The physician (PH) may thus view display device (27) to observe the real time positioning and shape, including any deformation, of the end effector in relation to the mapped aberrant conductive tissue sites and in relation to images of the adjacent anatomical structures in the patient (PA).

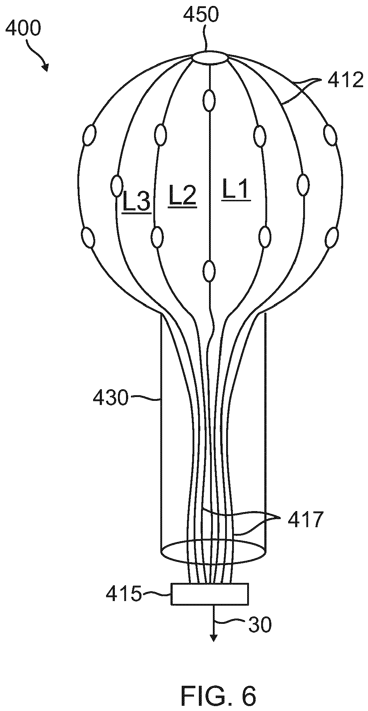

In some embodiments, splines may be arranged to define any suitably shaped basket end effector (400), such as a spheroidal basket shown in **FIG. 6. FIG. 6** shows an embodiment in which splines 412 electrically-conductive elements are printed onto the splines. For example, each of the conductive elements may comprise electrically-conductive paint that is painted on the splines. In other embodiments, the conducting elements comprise wires that are affixed to the surface of the splines. In yet other embodiments, the splines themselves are constructed of electrically-conductive material.

As part of its structural framework, the basket end effector (400) includes a plurality of splines (412) whose proximal ends extend from a proximal coupling member (430) and whose distal ends converge at a hub (450). Because each spline is connected to every other spline via the hub (450), a combination of any two selected splines with their respective electrically-conductive elements can form an EM loop (e.g., L1, L2, L3) where the voltage difference induced by the magnetic field(s) across the first and second conducting elements forming the loop can be measured at a connection point (430) located on the proximal coupling member. Notably, each loop (L1, L2, L3) need not be identical in shape and size to each other in that the pair of splines forming each loop need not be adjacent splines. An amplifier switch box (415) proximally of the basket end effector (400) receiving voltage signals via leads (417) can be configured to amplify the voltage difference between the selected splines of a loop and provide outputs to the processor of the console (12) via the cable (30), where such outputs advantageously indicate position and/or shape of the selected loop. With a plurality of loops, each in a different position and/or location, coverage of the structure of the basket end effector (400) with a sufficient plurality of EM loops on the surface, each in a different position or location on the end effector, enables the processor to monitor and track not only the position and orientation but the overall shape of the basket end effector (400) and any deformation it undergoes.

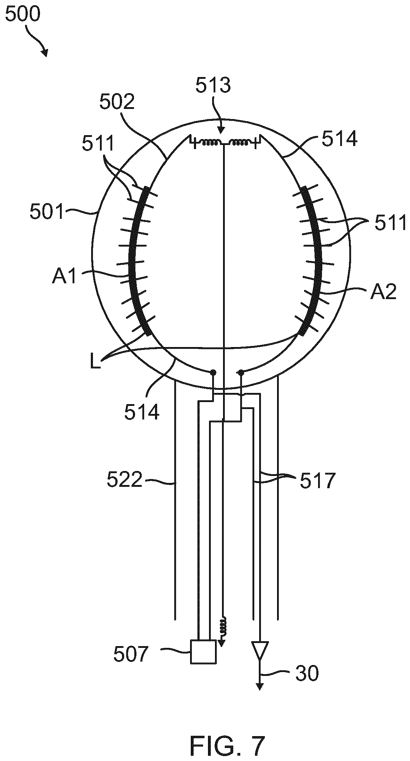

With reference to **FIG. 7****,** in some embodiments, a balloon end effector (500) has as electrically-conductive elements a flexible printed circuit board (PCB) (502) affixed to an outer surface (501) where the PCB (502) provides arrays (A1, A2) of electrodes (511) that are connected by leads (514) which form an EM loop (L). Signal outputs of the loop (L) are received by the processor of the console (12) via leads (517) extending proximally through catheter shaft (522) that are coupled to the cable (30), where the processor analyses the output for position and/or shape of the loop (L). In some embodiments, the EM loop (L) includes a resistor (513) between the two arrays (A1, A2) which allows the electrodes (511) of the arrays to also function as ablation electrodes, as energized by, for example, an RF generator (507) connected proximally of the balloon end effector (500).

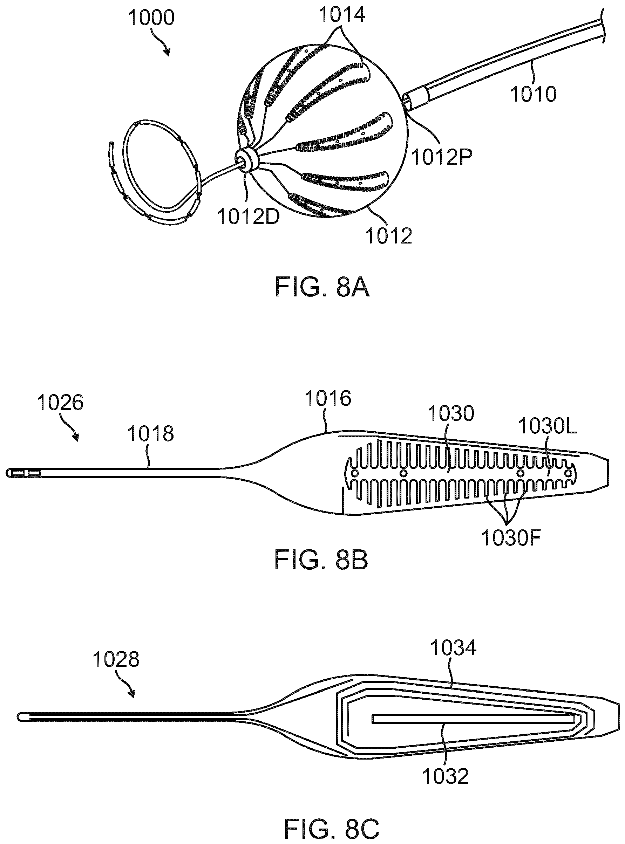

With reference to **FIG. 8A, FIG. 8B** and **FIG. 8C****,** in some embodiments, a catheter (1000) includes a shaft (1010) and a deformable balloon (1012), configured with a plurality of flexible printed circuit boards (PCBs) (1014) disposed on an outer surface of the balloon (1012). In the illustrated embodiment, each PCB (1014) is configured with a distal "leaf' portion (1016) and a proximal "stem" portion (1018) that lies along a respective longitudinal of the balloon (1012) to extend between distal and proximal ends (1022D, 1024P) of the balloon (1012). Each PCB (1014) includes a first or outer substrate (1026) with one or more electrical/electromagnetic sensors and a second or inner substrate (1028) with one or more electrical/electromagnetic sensors. In some embodiments, the outer layer (1026) provides one or more electrodes configured for tissue proximity indications (TPI) via impedance measurements, as described in U.S. Publication 2023/0028867, titled Accurate Tissue Proximity; U.S. Publication 2022/0183748A1, titled Accurate Tissue Proximity; U.S. Publication 2020/0177504A1, titled Tissue Proximity Indication Based on a Subset of Electrodes; and U.S. Publication 2020/0129089, titled Combined Active Current Location (ACL) and Tissue Proximity Indication (TPI) System, all contents of which are incorporated herein by reference.

In the illustrated embodiment, the first layer (1026) provides on an outer-facing surface an outer trace electrode (1030) and the second layer (1028) provides on an inner-facing surface an inner electrode (1032), together which are configured for impedance measurement for TPI. The trace electrode (1030) is configured as a "fishbone" with a longitudinal portion (1013L) and lateral finger portions (1030F). The lateral finger portions increase circumferential or equatorial contact surface of the trace electrode while gaps between adjacent finger portions allow the balloon to deflate/inflate. The inner electrode (1032) is situated below the longitudinal portion (1013L).

The second layer (1028) also provides one or more electromagnetic sensors that are configured as one or more EM loops (1034) that provide location orientation of the balloon (1012) and of balloon deformation in response to the magnetic field generators (32) of location pad (25) (FIG. 1). In some embodiments, balloon deformation (including inflation and deflation) as detected by the EM loop(s) is used to measure pressure change within the balloon in indicating, for example, balloon contact with tissue, for example, with a pulmonary vein ostium.

Electrical connections transmitting electrical signals to and from the trace electrode (1030), inner electrode (1032) and the EM loops (1034) extend along the respective stems portions (1018) of each layer (1026, 1028) of each PCB.

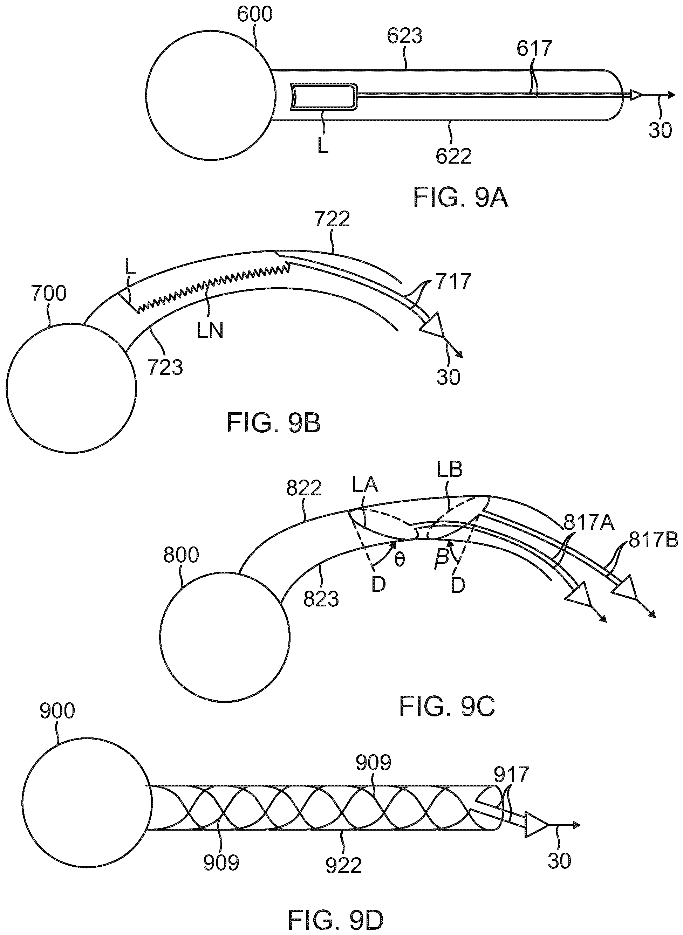

With reference to **FIG. 9A****,** in some embodiments, an EM loop (L) is configured to extend axially on an outer surface (623) of a catheter shaft (622) to detect bending or deformation of the shaft, especially at a location proximal of and adjacent to the end effector (600). The EM loop (L) is coupled to leads (617) such that EM loop signals are transmitted proximally through the shaft (622) and received by the processor of the console (12) via the cable (30).

With reference to **FIG. 9B****,** in some embodiments, an EM loop (L) is configured with one or more nonlinear segments (LN) on an outer surface (723) of a catheter shaft (722) so as to minimize mechanical strain when the catheter shaft is bent. The nonlinear segments (LN) configured, for example, as zig-zags, can accommodate stretching and/or compression of conductive element(s) of the EM loop when the shaft (722) is deflected or otherwise deformed, such as when an end effector (700) comes into contact with tissue. From the EM loop (L), leads (717) extend proximally through catheter shaft (722) and are coupled to the processor of the console (12) via the cable (30).

With reference to **FIG. 9C****,** in some embodiments, multiple EM loops (for example, loops LA, LB) are each configured circumferentially around an outer surface (823) of a catheter shaft (822), each with a respective pair of leads (817A, 817B) that extend proximally through the shaft and are coupled to the processor of the console (12) via the cable (30). In some embodiments, a plane defined by each EM loop is angularly off angularly offset from diameter D of the shaft (822) (see, for example, angle ⊖ and angle β). In some embodiments, the EM loops are overlapping, as shown in FIG. 8D, although it is understood that the EM loops may be nonoverlapping.

With reference to FIG. 9D, in some embodiments, a catheter shaft (922) has a sidewall (907) configured with embedded electrically-conductive element(s) (909), for example, electrically conductive wires (903) that extend continuous or electrically coupled in at least a portion of the shaft (922). The wires (903) may be configured in any suitable configuration, for example, in counter-clockwise, angularly-offset spirals extending distally and clockwise, oppositely angularly-offset spirals extending proximally, with ends that are coupled to the cable 30 for transmitting EM loop signals to the processor of the console (12). Coverage of the structure of the shaft (922) with a sufficient plurality of EM loops on the surface and/or in the interior of the shaft (922), each in a different position or location on the end effector, enables the processor to monitor and track not only the position and orientation but the overall shape of the shaft (922) and any deformation it undergoes.

Structural electrically-conductive materials such as the splines or embedded wires may include, for example, copper, stainless steel, and nitinol. Materials having shape memory may be advantageous in maintaining contact between electrodes carried on the splines and tissue surface.

After the processor of the console (12) analyzes the voltage measurement signals generated by the one or more EM loops on the end effector, shaft and/or the interface of the catheter, the system obtains apparent coordinates representing an apparent shape of any of these catheter elements in a local coordinate system. The processor may then apply a model of the mechanical properties of any of these catheter elements to the apparent coordinates. The processor may then compute a cost function with respect to shapes that can be assumed by any of these catheter elements within the patient's body. The processor may then identify and select a shape for any of these deformed catheter element in response to the cost function. The processor may then generate a set of corrected coordinates representing a corrected shape of any of these catheter elements. The processor using the corrected coordinates may drive the display device (27) to display a more accurate representation of any of these catheter elements. In this regard, the processor may apply a catheter mechanics algorithm that defines a catheter's parameter by combining characteristics known in advance, such as its neutral shape, bendability and/or flexibility, the dimensions of any of these catheter elements. Such a catheter mechanics algorithm is described in U.S. Publication No. 2021/0345902, titled "Catheter Shape and Position Detection Using Flexible Magnetic Sensor," the entire disclosure of which is incorporated herein by reference.

The preceding description has been presented with reference to presently preferred embodiments of the invention. Workers skilled in the art and technology to which this invention pertains will appreciate that alterations and changes in the described structure may be practiced without meaningfully departing from the principal, spirit and scope of this invention. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

Aspects of the invention:
1. An intrabody probe comprising:
   a deformable portion; and
   a flexible electromagnetic loop positioned on the deformable portion, the flexible electromagnetic loop configured to generate an induced voltage in response to an external magnetic field.
2. The intrabody probe of aspect 1, further comprising a distal electrode assembly and the deformable portion includes a deformable portion of the distal electrode assembly.
3. The intrabody probe of aspect 1, further comprising a shaft and the deformable portion includes a portion of a shaft.
4. The intrabody probe of aspect 1, further comprising a distal electrode assembly, a shaft, and an interface therebetween, and the deformation includes a portion of the interface.
5. The intrabody probe of aspect 1, wherein the flexible electromagnetic loop includes an electrically conductive spine.
6. The intrabody portion of aspect 1, wherein the flexible electromagnetic loop includes an electrically-conductive coating.
7. The intrabody portion of aspect 1, wherein the flexible electromagnetic loop includes an electrical trace.
8. The intrabody portion of aspect 1, wherein the flexible electromagnetic loop includes a flex circuit.
9. The intrabody probe of aspect 1, wherein the flexible electromagnetic loop is configured to generate a different induced voltage when the deformable portion experiences a deformation that changes the flexible magnetic loop from a neutral configuration into a deformed configuration.
10. The intrabody portion of aspect 9, wherein the induced voltage is indicative of a location of the flexible electromagnetic loop.
11. The intrabody portion of aspect 1, wherein the flexible electromagnetic loop is configured to generate the induced voltage in response to a first external magnetic field at a first frequency and to generate a second induced voltage in response to a second external magnetic field at a second frequency.
12. An intrabody probe comprising:
   a deformable portion; and
   a plurality of flexible electromagnetic loops, each in a different position on the deformable portion, each flexible electromagnetic loop configured to generate a respective induced voltage in response to an external magnetic field.
13. The intrabody probe of aspect 12, wherein at least one of the flexible electromagnetic loop includes an electrically conductive spine.
14. The intrabody probe of aspect 12, wherein at least one of the flexible electromagnetic loop includes an electrically-conductive coating.
15. The intrabody probe of aspect 12, wherein at least one of the flexible electromagnetic loop includes an electrical trace.
16. The intrabody probe of aspect 12, wherein at least one of the flexible electromagnetic loop includes a flex circuit.
17. The intrabody probe of aspect 12, wherein the flexible electromagnetic loops are configured to generate induces voltages indicative of locations of the loops in a local coordinate system.
18. The intrabody probe of aspect 12, wherein the flexible electromagnetic loops are configured to generate induced voltages indicative of deformations of the loops in a local coordinate system when the deformable portion transitions from a neutral configuration to a deformed configuration.
19. The intrabody probe of aspect 17, wherein each of the plurality of flexible electromagnetic loops is configured to generate the respective induced voltage in response to a first external magnetic field at a first frequency and to generate a respective second induced voltage in response to a second external magnetic field at a second frequency.
20. The intrabody probe of aspect 17, wherein each of the plurality of flexible electromagnetic loops is configured to generate a respective induced voltage when subjected to an external force that causes deformation of the respective loop, and a collection of the respective induced voltages of the plurality of loops is representative of a deformed shape of the deformable portion.
21. An intrabody probe comprising:
   a deformable portion;
   a first electrically-conductive element and a second electrically-conductive element, the first and second electrically-conductive elements configured as a flexible electromagnetic loop on the deformation portion;
      a first terminal of the first electrically-conductive element configured to carry a first induced voltage when exposed to a magnetic field;
   a second terminal of the second electrically-conductive element configured to carry a second induced voltage when exposed to the magnetic field; and
   a connection point configured to provide a voltage difference between the first and second induced voltages.
22. The intrabody probe of aspect 21, wherein the probe includes an electrophysiology catheter with a flexible end effector, the flexible end effector including the deformable portion.
23. The intrabody probe of aspect 21, wherein the probe includes an electrophysiology catheter with a flexible shaft, the flexible shaft including the deformable portion.
24. The intrabody probe of aspect 21, wherein the end effector includes first and second splines are configured as the first and second electrically-conductive elements.
25. The intrabody probe of aspect 21, wherein the first and second electrically-conductive elements include first and second electrically-conductive coatings on the end effector.
26. The intrabody probe of aspect 22, wherein the intrabody probe further comprises a flexible printed circuit board affixed to the flexible end effector, the printed circuit board including the first and second electronically-conductive elements.
27. The intrabody probe of aspect 21, wherein the first and second electrically-conductive elements are affixed to an outer surface of the shaft.
28. The intrabody probe of aspect 23, the shaft includes a sidewall and the first and second electrically-conductive elements include first and second wires embedded in the sidewall.
29. The intrabody probe of aspect 28, wherein the first electrically-conductive element includes a nonlinear segment.
30. The intrabody probe of aspect 28, wherein the first electrically-conductive element extends circumferentially about the shaft.
31. A system comprises:
   a magnetic field generator configured to generate a magnetic field;
   a catheter comprising:
      a deformable portion;
      a plurality of flexible electromagnetic loops, each of the plurality of flexible electromagnetic loops is affixed to a different position on the deformation portion, with each loop including a first electrically-conductive element, a second electrically-conductive element, a first terminal of the first electrically-conductive element configured to carry a first induced voltage when exposed to the magnetic field, and a second terminal of the second electrically-conductive element configured to carry a second induced voltage when exposed to the magnetic field
      at least a connection point configured to provide a voltage difference between the first and second induced voltages of each loop;
   a display, and
   a processor configured to:
      (i) receive the voltage difference of each loop from the at least one connection point;
      (ii) determine coordinates of each loop relative to a local coordinate system; and
      (iii) drive the display to display a shape as a visual representation of the deformable portion based on a collection of the coordinates of each loop.
32. The system of aspect 31, wherein the deformable portion includes electrodes, and the system further includes an RF generator configured to energize the electrodes for ablation of tissue.
33. The system of aspect 31, wherein the deformable portion includes a flexible end effector.
34. The system of aspect 31, wherein the deformable portion includes a flexible shaft.
35. The system of aspect 31, wherein the deformable portion includes an end effector with first and second splines that are configured as the first and second electrically-conductive elements.
36. The system of aspect 31, wherein the first and second electrically-conductive elements include first and second electrically-conductive coatings on the end effector.
37. The system of aspect 31, wherein the system further comprises a flexible printed circuit board affixed to the end effector, and the printed circuit board includes the first and second electronically-conductive elements.
38. The system of aspect 31, wherein the first and second electrically-conductive elements are affixed to an outer surface of the shaft.
39. The system of aspect 34, wherein the shaft includes a sidewall and the first and second electrically-conductive elements include first and second wires embedded in the sidewall.
40. The system of aspect 31, wherein the display displays the shape as a visual representation in real-time.
41. A system comprising:
   a magnetic field generator generating a magnetic field, and a catheter. The catheter comprises a deformable portion, a plurality of flexible electromagnetic loops, and at least a first connection point. Each of the plurality of flexible electromagnetic loops is affixed to the deformation portion at a different position, each loop including a first electrically-conductive element, a second electrically-conductive element, a first terminal of the first electrically-conductive element configured to carry a first induced voltage when exposed to the magnetic field, and a second terminal of the second electrically-conductive element configured to carry a second induced voltage when exposed to the magnetic field. The at least a connection point is configured to provide a voltage difference between the first and second induced voltages of each loop. The system further comprises a display, and a processor that is configured to (i) receive the voltage difference of each loop from the at least one connection point; (ii) determine coordinates of each loop relative to a local coordinate system; (iii) identify a shape for the deformable portion based on the coordinates of each loop; and (iv) drive the display to display the shape as a visual representation of the deformable portion.

## Claims

1. An intrabody probe comprising:
a deformable portion; and
a flexible electromagnetic loop positioned on the deformable portion, the flexible electromagnetic loop configured to generate an induced voltage in response to an external magnetic field.

2. The intrabody probe of claim 1, further comprising a distal electrode assembly and the deformable portion includes a deformable portion of the distal electrode assembly.

3. The intrabody probe of claim 1, further comprising a shaft and the deformable portion includes a portion of a shaft.

4. The intrabody probe of claim 1, further comprising a distal electrode assembly, a shaft, and an interface therebetween, and the deformation includes a portion of the interface.

5. The intrabody probe of claim 1, wherein the flexible electromagnetic loop includes at least one from the group of an electrically conductive spine, an electrically-conductive coating, an electrical trace, and a flex circuit.

6. The intrabody probe of claim 1, wherein the flexible electromagnetic loop is configured to generate a different induced voltage when the deformable portion experiences a deformation that changes the flexible magnetic loop from a neutral configuration into a deformed configuration, optionally, wherein the induced voltage is indicative of a location of the flexible electromagnetic loop.

7. The intrabody portion of claim 1, wherein the flexible electromagnetic loop is configured to generate the induced voltage in response to a first external magnetic field at a first frequency and to generate a second induced voltage in response to a second external magnetic field at a second frequency.

8. An intrabody probe comprising:
a deformable portion; and
a plurality of flexible electromagnetic loops, each in a different position on the deformable portion, each flexible electromagnetic loop configured to generate a respective induced voltage in response to an external magnetic field.

9. The intrabody probe of claim 8, wherein at least one of the flexible electromagnetic loops includes at least one from the group consisting of an electrically conductive spine., an electrically-conductive coating, an electrical trace, and a flex circuit.

10. The intrabody probe of claim 8, wherein the flexible electromagnetic loops are configured to generate induces voltages indicative of locations of the loops in a local coordinate system.

11. The intrabody probe of claim 8, wherein the flexible electromagnetic loops are configured to generate induced voltages indicative of deformations of the loops in a local coordinate system when the deformable portion transitions from a neutral configuration to a deformed configuration.

12. The intrabody probe of claim 11, wherein i) each of the plurality of flexible electromagnetic loops is configured to generate the respective induced voltage in response to a first external magnetic field at a first frequency and to generate a respective second induced voltage in response to a second external magnetic field at a second frequency, or ii) each of the plurality of flexible electromagnetic loops is configured to generate a respective induced voltage when subjected to an external force that causes deformation of the respective loop, and a collection of the respective induced voltages of the plurality of loops is representative of a deformed shape of the deformable portion.

13. An intrabody probe comprising:
a deformable portion;
a first electrically-conductive element and a second electrically-conductive element, the first and second electrically-conductive elements configured as a flexible electromagnetic loop on the deformation portion;
a first terminal of the first electrically-conductive element configured to carry a first induced voltage when exposed to a magnetic field;
a second terminal of the second electrically-conductive element configured to carry a second induced voltage when exposed to the magnetic field; and
a connection point configured to provide a voltage difference between the first and second induced voltages.

14. The intrabody probe of claim 13, wherein the probe includes an electrophysiology catheter with a flexible end effector, the flexible end effector including the deformable portion.

15. The intrabody probe of claim 13, wherein the probe includes an electrophysiology catheter with a flexible shaft, the flexible shaft including the deformable portion.
